# FASCICULE DE BREVET EUROPEEN

(11) **EP 1 433 467 B1**
(45) Date de publication et mention de la délivrance du brevet: **05.07.2006**
(21) Numéro de dépôt: 03293326.9
(22) Date de dépôt: 24.12.2003
(51) Int. Cl.: A61K 8/46, A61K 8/02, A61K 8/41, A61Q 5/04

(54) **Composition réductrice pour la mise en forme permanente ou le défrisage des cheveux contenant une phase mésomorphe, son procédé de préparation et procédé de mise en forme permanente ou de défrissage des cheveux**
Reduzierende Zusammensetzung zur dauerhaften Haarverformung und zur Glätten des Haares auf Basis einer mesomorphen Phase, ein Verfahren zur ihrer Herstellung und ein Verfahren zur dauerhaften Haarverformung und zur Glätten des Haares
Reducing composition for permanent waving or straightening of the hair comprising a mesomorphe phase, a process for its preparation and a process for the permanent waving or straightening of the hair

(30) Priorité: 24.12.2002 FR 0216597
(43) Date de publication de la demande: 30.06.2004
(73) Titulaire: L'ORÉAL, 75008 Paris (FR)
(72) Inventeur: Nicolas-Morgantini, Luc, 60810 Rully (FR); Petit, Jean-Marc, 92110 Clichy (FR)
(74) Mandataire: Casalonga, Axel

(56) Documents cités:
- WO-A-97/04738
- FR-A- 1 313 557
- FR-A- 2 816 210
- GB-A- 2 026 052
- US-A- 3 975 515
- US-A- 4 394 520
- US-A- 4 548 811
- US-A- 4 560 554
- US-A- 4 781 724
- US-B1- 6 173 717

## Description

La présente invention concerne une composition réductrice pour la mise en forme permanente ou le défrisage des cheveux, son procédé de préparation, un procédé de mise en forme permanente ou de défrisage ainsi qu'un dispositif à plusieurs compartiments ou "kit".

L'une des techniques couramment utilisée dans le domaine cosmétique pour donner aux cheveux une forme durable, consiste à procéder à la déformation des cheveux mettant en oeuvre un composé réducteur puis un composé oxydant.

La technique la plus usuelle pour obtenir une déformation permanente des cheveux consiste dans un premier temps à réaliser l'ouverture des liaisons disulfures (-S-S-) de la kératine (cystine) à l'aide d'une composition contenant un composé réducteur, puis après avoir de préférence rincé la chevelure ainsi traitée, à reconstituer dans un second temps lesdites liaisons disulfures en appliquant sur les cheveux préalablement mis sous tension par des bigoudis ou autres moyens, ou mis en forme ou lissés par d'autres, une composition oxydante encore appelée "fixateur" de façon à donner la forme recherchée aux cheveux.

Cette technique permet ainsi de réaliser, soit l'ondulation des cheveux, soit leur défrisage ou leur décrêpage ou encore leur lissage.

Cette nouvelle forme imposée aux cheveux par un traitement chimique, est durable dans le temps pendant quelques semaines et résiste notamment à l'action des lavages à l'eau ou aux shampoings et ceci par opposition aux techniques mettant en oeuvre des produits de coiffage entraînant une déformation temporaire, telle que la mise en plis, une telle déformation disparaissant au coiffage ou au shampoing.

Les compositions réductrices généralement utilisées pour la première étape d'une opération de mise en forme permanente ou de défrisage contiennent au moins un composé réducteur dans un milieu aqueux, et généralement de l'ammoniac comme agent alcalinisant.

Toutefois, ces compositions réductrices présentent un inconvénient particulier. En effet, lors de leur application sur les cheveux se produit un dégagement d'ammoniac entraînant une odeur désagréable.

La Demanderesse a trouvé de manière surprenante et inattendue que l'on pouvait limiter ce dégagement d'ammoniac de manière importante en ajoutant une phase mésomorphe dans une composition réductrice de mise en forme permanente ou de défrisage des cheveux comprenant au moins un composé réducteur et de l'ammoniac, en une quantité d'au moins 10 % en poids par rapport au poids total de ladite composition, ladite phase mésomorphe comprenant au moins un tensioactif non-ionique présantant une HBL inférieure ou égale à 10, et ledit ou lesdits tensioactifs non-ionique étant présent en une quantité allant de 5 à 30% en poids par rapport au poids total de la composition réductrice.

On obtient alors une limitation importante du dégagement d'ammoniac lors de l'application sur les cheveux, d'une composition réductrice de mise en forme permanente ou de défrisage, l'ammoniac étant contenu soit dans la phase mésomorphe soit à l'extérieur de cette phase mésomorphe.

La présente invention a donc pour objet une composition réductrice pour une mise en forme permanente ou un défrisage des cheveux comprenant, dans un milieu aqueux, au moins un composé réducteur, de l'ammoniac et au moins 10 % de phase mésomorphe par rapport au poids total de ladite composition.

L'invention concerne également un procédé de préparation de ladite composition.

Un autre objet de l'invention consiste en un procédé de mise en forme permanente et de défrisage des cheveux mettant en oeuvre ladite composition.

L'invention a encore pour objet un dispositif à plusieurs compartiments ou kit comprenant ladite composition.

D'autres objets, caractéristiques, aspects et avantages de l'invention apparaîtront encore plus clairement à la lecture de la description et des divers exemples qui suivent.

De préférence, la composition réductrice selon l'invention, pour la mise en forme permanente ou le défrisage des cheveux, comprend, dans un milieu aqueux, au moins un composé réducteur, de l'ammoniac, et au moins une phase mésomorphe en une quantité d'au moins 10 % en poids, mieux encore d'au moins 15 % en poids par rapport au poids de la composition.

De préférence, ladite composition comprend la phase mésomorphe en une quantité inférieure ou égale à 85 % en poids de la composition réductrice

La limitation significative du dégagement d'ammoniac lors de l'utilisation de la composition selon l'invention est observée que l'ammoniac se trouve dans la phase mésomorphe ou à l'extérieur de cette phase.

Par phase mésomorphe, on entend un état intermédiaire entre un état cristallin et un état liquide. La phase mésomorphe utilisée dans la composition selon l'invention est de préférence choisie parmi les phases hexagonales inverses (H₂), lamellaires (L_{α} et L_{β}) et cubiques inverses (I₂ et V₂). On utilise de préférence dans l'invention, une phase lamellaire L_{β}.

Par phase hexagonale inverse (H₂), on entend un arrangement hexagonal de micelles cylindriques parallèles de molécules amphiphiles (Handbook of lipid research 4, the physical chemistry of lipids from alkanes to phospholipids, D. M. Small Editor, 1986, Plenum Press, p. 51-56).

Par phase lamellaire fluide ou phase _{α}, on entend une phase dans laquelle les molécules de tensioactifs et/ou plus généralement de composés amphiphiles s'organisent sous forme de couches bimoléculaires espacées par des feuillets aqueux. Au sein des couches bimoléculaires, les molécules sont réparties selon une géométrie hexagonale ou orthorhombique et leurs chaînes grasses sont dans un état liquide, elles sont orientées perpendiculairement au plan des couches bimoléculaires mais n'ont pas d'orientation spécifique les unes par rapport aux autres dans le plan de ces couches (Handbook of lipid research 4, the physical chemistry of lipids from alkanes to phospholipids, D. M. Small Editor, 1986, Plenum Press, p. 51-56).

Par phase lamellaire gel ou phase L_{β}, on entend une phase dans laquelle les molécules de tensioactifs et/ou plus généralement de composés amphiphiles s'organisent sous forme de couches bimoléculaires espacées par des feuillets aqueux. Au sein des couches bimoléculaires, les molécules sont réparties selon une géométrie hexagonale ou orthorhombique et leurs chaînes hydrocarbonées sont dans un état cristallin, elles sont orientées perpendiculairement au plan des couches bimoléculaires mais n'ont pas d'orientation spécifique les unes par rapport aux autres dans le plan de ces couches (Handbook of lipid research 4, the physical chemistry of lipids from alkanes to phospholipids, D. M. Small Editor, 1986, Plenum Press, p. 51-56).

Par phase cubique, on entend une phase organisée de manière bipolaire en domaines hydrophile et lipophile distincts, en contact étroit et formant un réseau tridimensionnel à symétrie cubique. Une telle organisation a été notamment décrite dans "La Recherche", Vol. 23, pages 306-315, mars 1992 et dans "Lipid Technology", Vol. 2, n° 2, pages 42-45, avril 1990. Selon l'arrangement des domaines hydrophile et lipophile, la phase cubique est dite de type direct ou inverse. La phase cubique inverse correspond à une phase continue huileuse.

La structure de phase mésomorphe peut être vérifiée par microscopie de polarisation et diffusion de rayons X aux petits angles ou toute autre méthode bien connue dans la technique.

Selon la présente invention, la phase mésomorphe comprend au moins un tensioactif non ionique, de préférence mono- ou polyoxyalkyléné ou mono- ou polyglycérolé, présentant une HLB inférieure ou égale à 10, de préférence comprise entre 1 et 5.

La HLB ou balance hydrophile-lipophile du ou des tensioactifs non ioniques utilisés selon l'invention est la HLB selon GRIFFIN définie dans la publication J. Soc. Cosm. Chem. 1954 (Volume 5), pages 249-256, ou la HLB déterminée par voie expérimentale et telle que décrite dans l'ouvrage des auteurs F. PUISIEUX et M. SEILLER, intitulé "GALENICA 5 : Les systèmes dispersés - Tome I - Agents de surface et émulsions - Chapitre IV - Notions de HLB et de HLB critique, pages 153-194 - paragraphe 1.1.2. Détermination de HLB par voie expérimentale, pages 164-180.

Par tensioactifs non-ioniques mono- ou polyoxyalkylénés, on entend selon l'invention des tensioactifs non-ioniques qui portent dans leur molécule un ou plusieurs groupements oxyalkylène choisis parmi les groupements suivants -CH₂-CH₂-O-, -CH₂-CH₂-CH₂-O-, -CH₂₋CH(CH₃)-O- ou leurs mélanges

Par tensioactifs non-ioniques mono- ou polyglycérolés, on entend selon l'invention des tensioactifs non-ioniques qui portent dans leur molécule un ou plusieurs groupements glycérol.

Des exemples de tensioactifs utilisables dans la présente invention, sont les alcools gras polyglycérolés, les alcools gras mono- ou pblyoxyéthylénés, de préférence ayant plus de 2 moles d'oxyde d'éthylène, les amides gras mono- ou polyglycérolés comportant en moyenne de 1 à 5 groupements glycérol, les alkylphénols mono- ou polyoxyéthylénés, les condensats d'oxydes d'éthylène et de propylène sur des alcools gras, les huiles végétales polyoxyéthylées, de préférence ayant plus de 5 moles d'oxyde d'éthylene, les esters d'acides gras et de polyéthylèneglycols, les esters d'acides gras et de sorbitol polyoxyéthylénés, et leurs mélanges.

A titres d'exemples de tensioactifs non ioniques présentant une HLB > 5, on peut notamment citer ceux vendus sous les marques commerciales suivantes :

Imbentin POA/024 (HLB=5,5) par la société ICI, Synperonic PE L92 (HLB= 5,5) par la société ICI, Mergital LM2 (HLB=5,8) par la société HENKEL, Atlas G-70140 (HLB=6) par la société ICI, Imbentin. AG/124S/ 020 (HLB=6) par la société KOLB, Imbentin. L/125/025 HLB=6 par la société KOLB, Simulsol 989 (HLB=6) par la société SEPPIC, Soprophor HR10 (HLB=6) par la société RHONE POULENC, Kotilen O/1/050 (HLB=6,2)par la société KOLB, Croduret 10 (HLB=6,3) par la société CRODA, Etocas 10 (HLB=6,3) par la société CRODA, Imbentin OA/030 (HLB=6,3) par la société KOLB, Soprophor 208 (HLB=6,9) par la société RHONE POULENC, Ethylan 172 (HLB=7) par la société HARCROS, Akyporox NP 40 (HLB=7,1) par la société CHEM-Y, Polychol 5 (HLB=7,3) par la société CRODA, Arlatone 985 (HLB=7,5) par la société ICI, Sandoxylate FOL4 (HLB=7,5) par la société SANDOZ, Radiasurf 7453 (HLB=7,8) par la société OLEOFINA, Prox-onic OA-1/04 (HLB=7,9) par la société PROTEX, Prox-onic TD-1/03 (HLB=7,9) par la société PROTEX, Genapol PF 40 (HLB=8) par la société HOECHST, PGE-400 - DS (HLB=8) par la société HEFTI, PGE-400- DO (HLB=8) par la société HEFTI, Sapogenat 6-040 (HLB=8) par la société HOECHST, Intrasol FA28/50/4 (HLB=8,1) par la société STOCKHAUSEN, Serdox NOG 200 S (HLB=8,5) par la société SERVO, Berol 26 (HLB=8,9) par la société BEROL NOBEL, Genapol O-050 (HLB=9) par la société HOECHST, Prox-onic LA-1/04 (HLB=9,2) par la société PROTEX, Eumulgin O5 (HLB=9,5) par la société HENKEL, Etocas 20 (HLB=9,6) par la société CRODA, Antarox CO 520 (HLB=10) par la société RHONE POULENC, Imbentin POA/060 (HLB=10) par la société KOLB, TO-55-EL (HLB=10) par la société HEFTI.

On peut également citer à titres d'exemples de tensioactif non ionique présentant une HLB ≤ 5, ceux vendus sous les marques commerciales suivantes :

Synperonic PE L121 (HLB=0,5) par la société ICI, Prox-Onic EP 4060-1 (HLB=1) par la société PROTEX, Synperonic PE L101 (HLB= 1) par la société ICI, Etocas 29 (HLB=1,7) par la société CRODA, Genapol PF 10 (HLB=2) par la société HOECHST, Synperonic PE L81 (HLB=2) par la société ICI, Prox-Onic EP 1090-1 (HLB=3) par la société PROTEX, Sinnopal DPN2 (HLB=3,3) par la société HENKEL, Antarox CA 210 (HLB=3,5) par la société RHONE-POULENC, Antarox O1P (HLB=3,5) par la société RHONE-POULENC, Alkasurf OP11 (HLB=3,6) par la société RHONE-POULENC, Triton X15 (HLB=3,6) par la société ROHM et HAAS, Alkasurf OP1 (HLB=3,6) par la société RHONE-POULENC, Arlacel 121 (HLB=3,8) par la société ICI, Prox-Onic HR ou HRH-05 (HLB=3,8) par la société PROTEX, Etocas 5 (HLB=3,9) par la société HOECHST, Genapol PF20 (HLB=4) par la société HOECHST, Imbentin N/7 A (HLB=4) par la société KOLB, Synperonic PE L122 (HLB=4) par la société ICI, Ethylan NP1 (HLB=4,5) par la société HARCROS, Imbentin N/020 (HLB=4,5) par la société KOLB, Kotilen O/3/020 (HLB=4,5) par la société KOLB, Synperonic PE L31 (HLB=4,5) par la société ICI, TO-55-A (HLB=4,5) par la société HEFTI, Alkasurf NP-1 (HLB=4,6) par la société RHONE-POULENC, Antarox CO 210 (HLB=4,6) par la société RHONE-POULENC, Prox-Onic NP-1 (HLB=4,6) par la société PROTEX, Rhodiasurf NP2 (HLB=4,6) par la société RHONE-POULENC, Soprophor BC2 (HLB=4,6) par la société RHONE-POULENC, Triton N17 (HLB=4,6) par la société ROHM et HAAS, Akyporox NP15 (HLB=4,7) par la société CHEM-Y, Texofor M2 (HLB=4,8) par la société RHONE-POULENC, Alkasurf SA2 (HLB=4,9) par la société RHONE-POULENC, Arlacel 989 (HLB=4,9) par la société ICI, Brij 72 (HLB=4,9) par la société ICI, Brij 92 (HLB=4,9) par la société ICI, Brij 93 (HLB=4,9) par la société ICI, Prox-Onic SA-1 ou 2/02 (HLB=4,9) par la société PROTEX, Simulsol 72 (HLB=4,9) par la société SEPPIC, Simulsol 92 (HLB=4,9) par la société SEPPIC, Volpo S-2 (HLB=4,9) par la société CRODA, Arlacel 581 (HLB=5,0) par la société ICI, Arlacel 582 (HLB=5,0) par la société ICI, Genapol 0-020 (HLB=5,0) par la société HOECHST, Imbentin POA/020 (HLB=5,0) par la société KOLB, et Mergital Q2 (HLB=5,0) par la société HENKEL.

Le tensioactif non ionique convenant particulièrement bien à l'invention est l'alcool hexadécylique à 2 moles de glycérol

Le ou les tensioactifs non ioniques sont présents en une quantité allant de 5 à 30 % en poids, mieux encore de 5,5 à 30 % en poids, et encore plus préférentiellement de 10 à 20 % en poids, par rapport au poids total de la composition réductrice.

La phase mésomorphe peut également comprendre en outre au moins un alcool gras comportant de 8 à 30 atomes de carbone, de préférence de 12 à 22 atomes de carbone.

A titre d'exemples d'alcools gras, on peut notamment citer l'hexadécanol.

L'alcool gras est de préférence présent en une quantité allant de 3 à 20 % en poids, mieux encore de 5 à 15 % en poids, par rapport au poids total de la composition réductrice.

L'ammoniac est notamment présent dans la composition réductrice selon l'invention, en une quantité de 0,01 à 4 % en poids, de préférence de 0,05 à 2 % en poids par rapport au poids total de la composition.

A titre de composés réducteurs de la kératine, on peut notamment citer des sulfites et/ou des bisulfites de métaux alcalins ou alcalino-terreux ou d'ammonium ou, de préférence, des thiols. Parmi ces derniers, ceux les plus couramment utilisés sont la cystéïne et ses divers dérivés (notamment la N-acétylcystéïne), la cystéamine et ses divers dérivés (notamment ses dérivés acylés en C₁-C₄ tels que la N-acétyl cystéamine ou la N-propionyl cystéamine), l'acide thiolactique et ses esters (notamment le monothiolactate de glycérol), l'acide thioglycolique ainsi que ses esters, notamment le monothioglycolate de glycérol ou de glycol, et le thioglycérol. On peut également mentionner les composés réducteurs suivants : les N-mercaptoalkylamides de sucres tels que le N-(mercapto-2-éthyl)gluconamide, l'acide β-mercaptopropionique et ses dérivés, l'acide thiomalique, la panthétéïne, les N-(mercaptoalkyl)ω-hydroxyalkylamides décrits dans la demande de brevet EP-A-354 835 et les N-mono- ou N,N-dialkylmercapto 4-butyramides décrits dans la demande de brevet EP-A-368 763, les aminomercaptoalkylamides décrits dans la demande de brevet EP-A-432 000 et les alkylaminomercaptoalkylamides décrits dans la demande de brevet EP-A-514 282, le mélange de thioglycolate d'hydroxy-2 propyle (2/3) et de thioglycolate d'hydroxy-2 méthyl-1 éthyle (67/33) décrit dans la demande de brevet FR-A-2 679 448.

Les composés réducteurs préférés sont l'acide thioglycolique et la cystéine.

Dans les compositions réductrices pour la mise en forme permanente ou le défrisage des cheveux selon l'invention, les composés réducteurs tels que mentionnés ci-dessus sont généralement présents en une concentration qui peut être comprise entre 0,1 et 30% en poids, et de préférence entre 1 et 20% en poids, mieux encore entre 5 et 20% en poids par rapport au poids total de la composition réductrice.

Les compositions réductrices selon l'invention peuvent contenir en outre des additifs usuels et classiques, seuls ou en mélanges, tels que des agents tensioactifs différents de ceux cités ci-dessus, de type non-ionique, anionique, cationique ou amphotère. Parmi ceux-ci, on peut notamment citer les alkylsulfates, les alkylbenzènesulfates, les alkyléthersulfatcs, les alkylsulfonates, les sels d'ammonium quaternaire, les alkylbétaïnes, les alcanolamides d'acides gras, les esters d'acides gras oxyéthylénés, ainsi que d'autres tensio-actifs non-ioniques du type hydroxypropyléther.

Lorsque la composition réductrice contient en plus des tensioactifs non ioniques présentant une HLB inférieure ou égale à 10, au moins un agent tensioactif, ce dernier est généralement présent à une concentration maximale de 30% en poids, et de préférence comprise entre 0,5 et 10% en poids, par rapport au poids total de la composition réductrice.

Dans le but d'améliorer les propriétés cosmétiques des cheveux ou encore d'en atténuer ou d'éviter leur dégradation, la composition réductrice peut également contenir un agent traitant de nature cationique, anioniquc, non-ionique ou amphotère.

Parmi les agents traitants particulièrement préférés, on peut notamment citer ceux décrits dans les demandes de brevets français Nos 2 598 613 et 2 470 596. On peut également utiliser comme agents traitants des silicones volatiles ou non, linéaires ou cycliques et leurs mélanges, les polydiméthylsiloxanes, les polyorganosiloxanes quaternisés tels que ceux décrits dans la demande de brevet français No 2 535 730, les polyorganosiloxanes à groupements aminoalkyles modifiés par des groupements alcoxycarbonylalkyles tels que ceux décrits dans le brevet US No 4 749 732, des polyorganosiloxanes tels que le copolymere polydiméthylsiloxane-polyoxyalkyle du type Diméthicone-Copolyol, un polydiméthylsiloxane à groupements terminaux stéaroxy-(stéaroxydiméthicone), un copolymère polydiméthyl-siloxanedialkylammonium acétate ou un copolymère polydiméthyl-siloxane polyalkylbétaïne décrits dans la demande de brevet britannique No 2 197 352, des polysiloxanes organo modifiés par des groupements mercapto ou mercaptoalkyles tels que ceux décrits dans le brevet français n° 1 530 369 et dans la demande de brevet européen No 295 780, ainsi que des silanes tels que le stéaroxytriméthylsilane.

La composition réductrice peut également contenir d'autres ingrédients traitants tels que des polymères cationiques tels que ceux utilisés dans les compositions de brevets français Nos 2 472 382 et 2 495 931, ou encore des polymères cationiques du type ionène tels que ceux utilisés dans les compositions du brevet luxembourgeois n° 83703, des aminoacides basiques (tels que la lysine, l'arginine) ou acides (tels que l'acide glutamique, l'acide aspartique), des peptides et leurs dérivés, des hydrolysats de protéines, des cires, des agents de gonflement et de pénétration ou permettant de renforcer l'efficacité du réducteur tels que le mélange SiO₂/PDMS (polydiméthylsiloxane), le diméthylisosorbitol, l'urée et ses dérivés, la pyrrolidone, les N-alkyl-pyrrolidones, la thiamorpholinone, les alkyléthers d'alkylèneglycol ou de dialkylène-glycol tels que par exemple le monométhyléther de propylèneglycol, le monométhyléther de dipropylèneglycol, le monoéthyléther de l'éthylèneglycol et le monoéthyléther du diéthylèneglycol, des alcanediols en C₃-C₆ tels que par exemple le propanediol-1,2 et le butanediol-1,2, l'imidazolidinone-2 ainsi que d'autres composés tels que l'acide panthothénique, des agents antichute, des agents antipelliculaires, des épaississants, des agents de suspension, des agents séquestrants, des agents opacifiants, des colorants, des filtres solaires ainsi que des parfums et des conservateurs.

La composition réductrice peut se présenter sous la forme d'une lotion, épaissie ou non, d'une crème, d'un gel, ou de toute autre forme appropriée.

Le milieu aqueux utilisé dans la présente invention est constitué de préférence par l'eau ou par une solution hydroalcoolique d'un alcool inférieur en C₁₋₄ tel que l'éthanol, l'isopropanol ou le butanol.

Un mode de réalisation particulièrement intéressant de l'invention consiste à utiliser un milieu aqueux ne contenant pas de solvant organique.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes sous forme d'émulsions « lourdes », dont la phase huileuse peut être constituée par divers produits tels que l'huile de paraffine, l'huile de vaseline, l'huile d'amande douce, l'huile d'avocat, l'huile d'olive, des esters d'acides gras comme le monostéarate de glycéryle, les palmitates d'éthyl ou d'isopropyle, les myristates d'alkyle tels que les myristates de propyle, de butyle ou de cétyle. On peut en outre ajouter des alcools gras comme l'alcool cétylique ou des cires telles que par exemple la cire d'abeille.

On peut également utiliser des liquides ou des gels contenant des agents épaississants tels que des polymères ou des copolymères carboxyvinyliques qui « collent » les cheveux et les maintiennent dans la position lisse pendant le temps de pose.

Enfin, les compositions réductrices peuvent également contenir au moins un disulfure connu pour son utilisation dans une composition réductrice pour permanente auto-neutralisante.

Parmi de tels disulfures connus, on peut notamment mentionner l'acide dithioglycolique, le dithioglycérol, la cystamine, la N,N'-diacétyl-cystamine, la cystine, la pantéthine, et les disulfures des N-(mercapto-alkyl)ω-hydroxyalkylamides décrits dans la demande de brevet européen EP 354 835, les disulfures des N-mono ou N,N-dialkylmercapto-4-butyramides décrits dans la demande de brevet EP 368 763, les disulfures des aminomercapto-alkylamides décrits dans la demande de brevet EP 432 000, et les disulfures des alkylaminomercaptoalkylamides décrits dans la demande de brevet EP 514 282. Ces disulfures sont généralement présents dans un rapport molaire de 0,5 à 2,5, et de préférence de 1 à 2, par rapport à l'agent réducteur (voir brevet US 3 768 490).

Le pH des compositions réductrices peut être éventuellement ajusté par ajout d'agents acidifiants tels que, par exemple, l'acide chlorhydrique, l'acide acétique, l'acide lactique ou l'acide borique, ou d'agents alcalinisants différents de l'ammoniac.

Le pH est généralement compris entre 6 et 10, de préférence entre 7 et 9,5.

La présente invention a également pour objet un procédé de préparation de la composition réductrice pour la mise en forme ou le défrisage des cheveux telle que définie ci-dessus. Il comprend les étapes de :
a) mélange d'au moins un tensioactif non ionique présentant une HLB inférieure ou égale à 10, de préférence comprise entre 1 et 5, avec de l'eau, et éventuellement avec un ou plusieurs alcools gras, pour préparer la phase mésomorphe, et
b) addition d'au moins un composé réducteur tel que décrit ci-dessus.

L'ammoniac est ajouté dans l'étape a) et/ou dans l'étape b), en d'autres termes, il est soit ajouté au moment de la préparation de la phase mésomorphe, soit déjà présent dans le milieu de la composition réductrice avant addition de la phase mésomorphe, soit ajouté dans la composition réductrice après incorporation de la phase mésomorphe.

Un autre mode de réalisation consiste à incorporer une phase mésomorphe contenant de l'ammoniac dans un milieu contenant lui-même de l'ammoniac.

Un autre objet de l'invention consiste en un procédé de mise en forme permanente ou de défrisage des cheveux comprenant les étapes consistant à :
appliquer une composition réductrice telle que définie ci-dessus, pendant ou après l'étape de mise sous tension des cheveux par un moyen mécanique ou mis en forme par tous les moyens manuels,
appliquer après un temps de pose suffisant pour permettre la réduction des liaisons disulfures des cheveux, et après un rinçage éventuel, une composition oxydante contenant au moins un agent oxydant ; et à
procéder au rinçage final.

L'étape de mise sous tension des cheveux peut être mise en oeuvre par tous moyens mécaniques appropriés et connus, tels que des rouleaux, des bigoudis.

Les agents oxydants utilisables dans le cadre de la présente invention peuvent notamment être choisis parmi le peroxyde d'hydrogène ou eau oxygénée, le peroxyde d'urée ; les bromates de métaux alcalins ; les persels tels que les perborates et persulfates ; et les enzymes telles que les peroxydases et les oxydo-réductases à deux électrons. L'utilisation du peroxyde d'hydrogène ou desdits bromates est particulièrement préférée.

La concentration d'eau oxygénée peut varier de 1 à 10 volumes, mais elle est de préférence égale à environ 8 volumes.

La concentration des bromates est généralement comprise entre 1 et 12 % en poids et celle des persels est généralement comprise entre 0,1 et 15 % en poids par rapport au poids total de la composition oxydante.

Le pH de la composition oxydante peut être comprise entre 2 et 7, et de préférence entre 3 et 6.

L'invention concerne également un dispositif à plusieurs compartiments ou "kit" pour la mise en forme permanente ou le défrisage des cheveux, comportant au moins deux compartiments dont l'un d'entre eux renferme une composition réductrice telle que définie ci-dessus.

Les exemples suivants sont donnés à titre illustratif de l'invention.

### EXEMPLES

### Exemple 1 : Préparation d'une phase mésomorphe lamellaire L_{β}

On a ajouté de l'eau et de l'ammoniac à un mélange d'alcool hexadécylique à 2 moles de glycérol (C₁₆G₂) et d'hexadécanol (C₁₆OH), les proportions étant indiquées dans le tableau 1 ci-dessous. On a fait subir à la structure trois cycles thermiques de deux heures, en faisant varier la température de la température ambiante à une température de l'ordre de 70 °C, sous agitation.

On a également préparé une composition aqueuse pour servir de témoin, ainsi qu'une dispersion de la phase lamellaire L_{β} dans de l'eau (ou dispersion L_{β}).

**Tableau 1**

| | % en poids | | | |
|---|---|---|---|---|
| | C₁₆G₂ | C₁₆OH | NH₃ | Eau |
| Composition aqueuse | - | - | 1 | 99 |
| Phase lamellaire L_{β} | 52,5 | 32,5 | 1 | 14 |
| Dispersion L_{β} | 10,5 | 6,5 | 1 | 82 |

On a vérifié la structure de la phase lamellaire par microscopie de polarisation et diffusion de rayons X aux petits angles.

On a ensuite mesuré le dégagement d'ammoniac avec des tubes colorimétriques, sur des échantillons de 10 ml de chacune des compositions, contenus dans des flacons de 30 ml, ouverts. L'incertitude résultante est d'environ 10 % au maximum. Les mesures pour chaque échantillon ont duré de 20 à 30 secondes.

| | Dégagement NH₃ (ppm) au bout de | | | |
|---|---|---|---|---|
| | 1 min. | 5 min. | 10 min. | 20 min. |
| Composition aqueuse | > 1400 | 1367 | 1300 | 1133 |
| Phase lamellaire L_{β} | 433 | 283 | 183 | 133 |
| Dispersion L_{β} | 533 | 333 | 367 | 350 |

L'échantillon de phase lamellaire diffuse de 3 à 8 fois moins que l'échantillon témoin.

La dispersion de phase lamellaire dans un excès d'eau présente un dégagement 3 à 4 fois inférieur à celui de l'échantillon témoin. On retrouve ce résultat que l'ammoniac soit présent dans la phase mésomorphe avant dispersion ou dans l'eau de dispersion.

### Exemple 2 :

On a préparé une composition réductrice en mélangeant à température ambiante (de l'ordre de 20 à 25 °C), 100 g. de phase lamellaire préparée dans l'exemple 1, et 15 g. d'un support de permanente dont les composants sont indiqués ci-dessous.

Les composants de la composition réductrice, y compris les composants de la phase lamellaire, sont indiqués ci-dessous avec leur proportion pondérale par rapport au poids total de la composition réductrice.

| Composants de la composition réductrice : | % en poids |
|---|---|
| - Bicarbonate d'ammonium | 5,3 |
| - Sel pentasodique de l'acide diéthylène-triamine-pentacétique en solution aqueuse à 40% | 0,17 |
| - Alcool oléocétylique oxyéthyléné (20 OE) | 0,67 |
| - Parfum | 0,33 |
| - Mélange cocoylamidopropylbétaine/monolaurate de glycéryle en solution aqueuse à 30% | 1,1 |
| - Polydiméthylsiloxane à groupes aminoéthyle, aminopropyle et alpha-oméga silanols, en émulsion cationique | 2,2 |
| - Thioglycolate d'ammonium en solution aqueuse à 71% | 12,8 |
| - Polycondensat tétraméthyl-hexaméthylènediamine/ 1,3-dichloropropylène en solution aqueuse à 60 % | 1,3 |
| - Ammoniaque (20%) | 2,0 |
| - Alcool hexadécylique à 2 moles de glycérol | 8,8 |
| - Hexadécanol | 5,4 |
| - Eau désionisée qs | 100 |

On constate une nette diminution de la quantité d'ammoniac dégagé au cours du temps.

## Revendications

1. Composition réductrice de mise en forme permanente ou de défrisage des cheveux, **caractérisée en ce qu'**elle comprend, dans un milieu aqueux, au moins un composé réducteur de la kératine, de l'ammoniac et au moins une phase mésomorphe présente en une quantité d'au moins 10 % en poids par rapport au poids de la composition, ladite phase mésomorphe comprenant au moins un tensioactif non-ionique présentant une HLB inférieure ou égale à 10, et ledit ou lesdits tensioactifs non-ioniques étant présent en une quantité allant de 5 à 30% en poids, par rapport au poids total de la composition réductrice.

2. Composition selon la revendication 1, **caractérisée en ce que** la quantité de phase mésomorphe est supérieure ou égale à 15 % en poids par rapport au poids de la composition.

3. Composition selon la revendication 1 ou 2, **caractérisée en ce que** la quantité de phase mésomorphe est inférieure ou égale à 85 % en poids par rapport au poids total de la composition.

4. Composition selon l'une quelconque des revendications 1 à 3, **caractérisée en ce que** la phase mésomorphe est choisie parmi les phases hexagonales inverses (H₂), lamellaires (L_{α} et L_{β}) et cubiques inverses (I₂ et V₂).

5. Composition selon la revendication 4, **caractérisée en ce que** la phase mésomorphe est une phase lamellaire L_{β}.

6. Composition selon l'une quelconque des revendications 1 à 5, **caractérisée en ce que** la phase mésomorphe comprend au moins un tensioactif non ionique présentant une HLB comprise entre 1 et 5.

7. Composition selon l'une quelconque des revendications précédentes, **caractérisée en ce que** les tensioactifs non ioniques sont choisis parmi les alcools gras polyglycérolés, les alcools gras mono- ou polyoxyéthylénés, les amides gras mono- ou polyglycérolés comportant en moyenne de 1 à 5 groupements glycérol, les alkylphénols mono- ou polyoxyéthylénés ayant plus de 2 moles d'oxyde d'éthylène, les condensats d'oxydes d'éthylene et de propylène sur des alcools gras, les huiles végétales polyoxyéthylénées ayant plus de 5 moles d'oxyde d'éthylène, les esters d'acides gras et de polyéthylèneglycols, les esters d'acides gras et de sorbitol polyoxyéthylénés, et leurs mélanges.

8. Composition selon la revendication 7, **caractérisée en ce que** le tensioactif non ionique est l'alcool hexadécylique à 2 moles de glycérol.

9. Composition selon l'une quelconque des revendications 1 à 8, **caractérisée en ce que** le tensioactif non ionique est présent en une quantité allant de 10 à 20 % en poids, par rapport au poids total de la composition réductrice.

10. Composition selon l'une quelconque des revendications précédentes, **caractérisée en ce que** la phase mésomorphe comprend en outre au moins un alcool gras comportant de 8 à 30 atomes de carbone.

11. Composition selon la revendication 10, **caractérisée en ce que** l'alcool gras est l'hexadécanol.

12. Composition selon la revendication 10 ou 11, **caractérisée en ce que** l'alcool gras est présent en une quantité allant de 3 à 20 % en poids, mieux encore de 5 à 15 % en poids, par rapport au poids total de la composition réductrice.

13. Composition selon l'une quelconque des revendications précédentes, **caractérisée en ce qu'**elle comprend l'ammoniac en une quantité de 0,01 à 4 % en poids par rapport au poids total de la composition

14. Composition selon la revendication 13, **caractérisée en ce qu'**elle comprend l'ammoniac en une quantité de 0,05 à 2 % en poids par rapport au poids total de la composition.

15. Composition selon l'une quelconque des revendications précédentes, **caractérisée en ce que** le composé réducteur est choisi parmi les sulfites et/ou les bisulfites de métaux alcalins ou alcalino-terreux ou d'ammonium, et les thiols.

16. Composition selon la revendication 15, **caractérisée en ce que** le composé réducteur est choisi parmi l'acide thioglycolique et la cystéine.

17. Composition selon l'une quelconque des revendications précédentes, **caractérisée en ce que** le composé réducteur est présent en une quantité comprise entre 1 et 30% en poids, et de préférence entre 5 et 20% en poids par rapport au poids total de la composition réductrice.

18. Composition selon l'une quelconque des revendications précédentes, **caractérisée en ce que** le milieu aqueux est constitué par l'eau ou par une solution hydroalcoolique d'un alcool inférieur en C₁₋₄ tel que l'éthanol, l'isopropanol ou le butanol.

19. Procédé de préparation de la composition réductrice selon l'une quelconque des revendications précédentes, **caractérisé en ce qu'**il comprend les étapes de :
a) mélange d'au moins un tensioactif non ionique présentant une HLB inférieure ou égale à 10, de préférence comprise entre 1 et 5, avec de l'eau, et éventuellement avec un ou plusieurs alcools gras, pour préparer la phase mésomorphe, et
b) addition d'au moins un composé réducteur, l'ammoniac étant ajouté dans l'étape a) et/ou l'étape b).

20. Procédé de mise en forme permanente ou de défrisage des cheveux, **caractérisé en ce que** l'on applique une composition réductrice selon l'une quelconque des revendications 1 à 18, pendant ou après l'étape de mise sous tension des cheveux par un moyen mécanique ou mis en forme par tous les moyens manuels,
l'on applique après un temps de pose suffisant pour permettre la réduction des liaisons disulfures des cheveux, et après un rinçage éventuel, une composition oxydante contenant au moins un agent oxydant, et
l'on procède au rinçage.

21. Procédé de mise en forme permanente ou de défrisage des cheveux selon la revendication 20, **caractérisé en ce que** l'agent oxydant est choisi parmi les bromates de métaux alcalins et le peroxyde d'hydrogène.

22. Dispositif à plusieurs compartiments ou "kit" pour la mise en forme permanente ou le défrisage des cheveux, comportant au moins deux compartiments dont l'un d'entre eux renferme une composition réductrice selon l'une quelconque des revendications 1 à 18.

## Claims

1. Reducing composition for permanently reshaping or relaxing the hair, **characterized in that** it comprises, in an aqueous medium, at least one keratin-reducing compound, ammonia and at least one mesomorphic phase present in an amount of at least 10% by weight relative to the weight of the composition, the said mesomorphic phase comprising at least one nonionic surfactant with an HLB value of less than or equal to 10, and the said nonionic surfactants being present in an amount ranging from 5% to 30% by weight relative to the total weight of the reducing composition.

2. Composition according to Claim 1, **characterized in that** the amount of mesomorphic phase is greater than or equal to 15% by weight relative to the weight of the composition.

3. Composition according to Claim 1 or 2, **characterized in that** the amount of mesomorphic phase is less than or equal to 85% by weight relative to the total weight of the composition.

4. Composition according to any one of Claims 1 to 3, **characterized in that** the mesomorphic phase is chosen from inverse hexagonal phases (H₂), lamellar phases (L_{α} and L_{β}) and inverse cubic phases (I₂ and V₂).

5. Composition according to Claim 4, **characterized in that** the mesomorphic phase is a lamellar phase L_{β}.

6. Composition according to any one of Claims 1 to 5, **characterized in that** the mesomorphic phase comprises at least one nonionic surfactant with an HLB value of between 1 and 5.

7. Composition according to any one of the preceding Claims, **characterized in that** the nonionic surfactants are chosen from polyglycerolated fatty alcohols, monooxyethylenated or polyoxyethylenated fatty alcohols, monoglycerolated or polyglycerolated fatty amides comprising on average from 1 to 5 glycerol groups, monooxyethylenated or polyoxyethylenated alkylphenols containing more than 2 mol of ethylene oxide, condensates of ethylene oxide and of propylene oxide with fatty alcohols, polyoxyethylenated plant oils containing more than 5 mol of ethylene oxide, fatty acid esters of polyethylene glycols, polyoxyethylenated fatty acid esters of sorbitol, and mixtures thereof.

8. Composition according to Claim 7, **characterized in that** the nonionic surfactant is hexadecyl alcohol containing 2 mol of glycerol.

9. Composition according to any one of Claims 1 to 8, **characterized in that** the nonionic surfactant is present in an amount ranging from 10% to 20% by weight relative to the total weight of the reducing composition.

10. Composition according to any one of the preceding claims, **characterized in that** the mesomorphic phase also comprises at least one fatty alcohol containing from 8 to 30 carbon atoms.

11. Composition according to Claim 10, **characterized in that** the fatty alcohol is hexadecanol.

12. Composition according to Claim 10 or 11, **characterized in that** the fatty alcohol is present in an amount ranging from 3% to 20% by weight and better still from 5% to 15% by weight relative to the total weight of the reducing composition.

13. Composition according to any one of the preceding claims, **characterized in that** it comprises ammonia in an amount of from 0.01% to 4% by weight relative to the total weight of the composition.

14. Composition according to Claim 13, **characterized in that** it comprises ammonia in an amount of from 0.05% to 2% by weight relative to the total weight of the composition.

15. Composition according to any one of the preceding claims, **characterized in that** the reducing compound is chosen from alkali metal or alkaline-earth metal or ammonium sulphites and/or bisulphites, and thiols.

16. Composition according to Claim 15, **characterized in that** the reducing compound is chosen from thioglycolic acid and cysteine.

17. Composition according to any one of the preceding claims, **characterized in that** the reducing compound is present in an amount of from 1% to 30% by weight, and preferably from 5% to 20% by weight relative to the total weight of the reducing composition.

18. Composition according to any one of the preceding claims, **characterized in that** the aqueous medium consists of water or of an aqueous-alcoholic solution of a C₁₋₄ lower alcohol such as ethanol, isopropanol or butanol.

19. Process for preparing the reducing composition according to any one of the preceding claims, **characterized in that** it comprises the steps of:
a) mixing at least one nonionic surfactant with an HLB value of less than or equal to 10, preferably between 1 and 5, with water and optionally with one or more fatty alcohols, to prepare the mesomorphic phase, and
b) adding at least one reducing compound,
the ammonia being added in step a) and/or step b).

20. Process for permanently reshaping or relaxing the hair, **characterized in that** a reducing composition according to any one of Claims 1 to 18 is applied, during or after the step of placing the hair under tension via a mechanical means or shaping via any manual means,
after a leave-in time that is sufficient to allow reduction of the disulphide bonds of the hair, and after optional rinsing, an oxidizing composition containing at least one oxidizing agent is applied, and
rinsing is performed.

21. Process for permanently reshaping or relaxing the hair according to Claim 20, **characterized in that** the oxidizing agent is chosen from alkali metal bromates and hydrogen peroxide.

22. Multi-compartment device or kit for permanently reshaping or relaxing the hair, comprising at least two compartments, one of which contains a reducing composition according to any one of Claims 1 to 18.

## Patentansprüche

1. Reduzierende Zusammensetzung für die dauerhafte Verformung oder Entkräuselung von Haaren, **dadurch gekennzeichnet, dass** sie in einem wässrigen Medium mindestens eine das Keratin reduzierende Verbindung, Ammoniak und mindestens eine mesomorphe Phase enthält, die in einem Mengenanteil von mindestens 10 Gew.-%, bezogen auf das Gesamtgewicht der Zusammensetzung, enthalten ist, wobei die mesomorphe Phase mindestens einen nichtionischen grenzflächenaktiven Stoff enthält, der einen HLB-Wert von 10 oder darunter aufweist, und der oder die nichtionische(n) grenzflächenaktive(n) Stoff(e) in einer Menge von 5 bis 30 Gew.-%, bezogen auf das Gesamtgewicht der reduzierenden Zusammensetzung, vorliegen.

2. Zusammensetzung nach Anspruch 1, **dadurch gekennzeichnet, dass** der Anteil der mesomorphen Phase 15 Gew.-% beträgt oder darüber liegt, bezogen auf das Gesamtgewicht der Zusammensetzung.

3. Zusammensetzung nach Anspruch 1 oder 2, **dadurch gekennzeichnet, dass** der Anteil der mesomorphen Phase höchstens 85 Gew.-%, bezogen auf das Gesamtgewicht der Zusammensetzung, beträgt.

4. Zusammensetzung nach einem der Ansprüche 1 bis 3, **dadurch gekennzeichnet, dass** die mesomorphe Phase unter den inversen hexagonalen Phasen (H₂), lamellaren Phasen (L_{α} und L_{β}) und inversen kubischen Phasen (I₂ und V₂) ausgewählt ist.

5. Zusammensetzung nach Anspruch 4, **dadurch gekennzeichnet, dass** die mesomorphe Phase eine lamellare Phase L_{β} ist.

6. Zusammensetzung nach einem der Ansprüche 1 bis 5, **dadurch gekennzeichnet, dass** die mesomorphe Phase mindestens einen nichtionischen grenzflächenaktiven Stoff enthält, der einen HLB-Wert von 1 bis 5 aufweist.

7. Zusammensetzung nach einem der vorhergehenden Ansprüche, **dadurch gekennzeichnet, dass** die nichtionischen grenzflächenaktiven Stoffe unter den mehrfach mit Glycerin veretherten Fettalkoholen, mono- oder polyethoxylierten Fettalkoholen, einfach oder mehrmals mit Glycerin veretherten Fettamiden, die im Mittel 1 bis 5 Glyceringruppen enthalten, mono- oder polyethoxylierten Alkylphenolen, die mehr als 2 mol Ethylenoxid enthalten, Kondensaten von Ethylenoxid und Propylenoxid mit Fettalkoholen, polyethoxylierten pflanzlichen Ölen mit mehr als 5 mol Ethylenoxid, Polyethylenglykolfettsäureestern, polyethoxylierten Sorbitfettsäureestern und deren Gemischen ausgewählt sind.

8. Zusammensetzung nach Anspruch 7, **dadurch gekennzeichnet, dass** der nichtionische grenzflächenaktive Stoff der Hexadecylalkohol mit 2 mol Glycerin ist.

9. Zusammensetzung nach einem der Ansprüche 1 bis 8, **dadurch gekennzeichnet, dass** der nichtionische grenzflächenaktive Stoff in einer Menge von 10 bis 20 Gew.-%, bezogen auf das Gesamtgewicht der reduzierenden Zusammensetzung, enthalten ist.

10. Zusammensetzung nach einem der vorhergehenden Ansprüche, **dadurch gekennzeichnet, dass** die mesomorphe Phase ferner mindestens einen Fettalkohol mit 8 bis 30 Kohlenstoffatomen enthält.

11. Zusammensetzung nach Anspruch 10, **dadurch gekennzeichnet, dass** es sich bei dem Fettalkohol um Hexadecanol handelt.

12. Zusammensetzung nach Anspruch 10 oder 11, **dadurch gekennzeichnet, dass** der Fettalkohol in einer Menge von 3 bis 20 Gew.-%, besser 5 bis 15 Gew.-%, bezogen auf das Gesamtgewicht der reduzierenden Zusammensetzung, vorliegt.

13. Zusammensetzung nach einem der vorhergehenden Ansprüche, **dadurch gekennzeichnet, dass** sie Ammoniak in einer Menge von 0,01 bis 4 Gew.-%, bezogen auf das Gesamtgewicht der Zusammensetzung, enthält.

14. Zusammensetzung nach Anspruch 13, **dadurch gekennzeichnet, dass** sie Ammoniak in einer Menge von 0,05 bis 2 Gew.-%, bezogen auf das Gesamtgewicht der Zusammensetzung, enthält.

15. Zusammensetzung nach einem der vorhergehenden Ansprüche, **dadurch gekennzeichnet, dass** das Reduktionsmittel unter den Sulfiten und/oder Bisulfiten von Alkalimetallen oder Erdalkalimetallen oder Ammonium und Thiolen ausgewählt ist.

16. Zusammensetzung nach Anspruch 15, **dadurch gekennzeichnet, dass** das Reduktionsmittel unter Thioglycolsäure und Cystein ausgewählt ist.

17. Zusammensetzung nach einem der vorhergehenden Ansprüche, **dadurch gekennzeichnet, dass** das Reduktionsmittel in einer Menge von 1 bis 30 Gew.-% und vorzugsweise 5 bis 20 Gew.-%, bezogen auf das Gesamtgewicht der reduzierenden Zusammensetzung, enthalten ist.

18. Zusammensetzung nach einem der vorhergehenden Ansprüche, **dadurch gekennzeichnet, dass** das wässrige Medium aus Wasser oder einer wässrig-alkoholischen Lösung eines niederen C₁₋₄₋Alkohols, wie Ethanol, Isopropanol oder Butanol, besteht.

19. Verfahren zur Herstellung der reduzierenden Zusammensetzung nach einem der vorhergehenden Ansprüche, **dadurch gekennzeichnet, dass** es die folgenden Schritte umfasst:
a) Mischen mindestens eines nichtionischen grenzflächenaktiven Stoffes, der einen HLB-Wert von 10 oder darunter und vorzugsweise im Bereich von 1 bis 5 aufweist, mit Wasser und gegebenenfalls mit einem oder mehreren Fettalkoholen zur Herstellung der mesomorphen Phase und
b) Zugabe mindestens eines Reduktionsmittels,
wobei das Ammoniak in Schritt a) und/oder Schritt b) zugegeben wird.

20. Verfahren zur dauerhaften Verformung oder Entkräuselung der Haare, **dadurch gekennzeichnet, dass** eine reduzierende Zusammensetzung nach einem der Ansprüche 1 bis 18 während des Schrittes, in dem die Haare mit mechanischen Mitteln unter Spannung gesetzt werden oder mit allen manuellen Mitteln in Form gebracht werden, oder nach diesem Schritt aufgetragen wird,
nach einer Einwirkzeit, die ausreichend ist, um die Disulfidbindungen der Haare zu reduzieren, und nachdem gegebenenfalls gespült wurde, eine oxidierende Zusammensetzung aufgebracht wird, die mindestens ein Oxidationsmittel enthält, und dann gespült wird.

21. Verfahren zur dauerhaften Verformung oder Entkräuselung der Haare nach Anspruch 20, **dadurch gekennzeichnet, dass** das Oxidationsmittel unter den Alkalimetallbromaten und Wasserstoffperoxid ausgewählt ist.

22. Vorrichtung mit mehreren Abteilungen oder "Kit" für die dauerhaften Verformung oder Entkräuselung der Haare, die mindestens zwei Abteilungen aufweist, wobei eine Abteilung eine reduzierende Zusammensetzung nach einem der Ansprüche 1 bis 18 enthält.
